# EUROPEAN PATENT SPECIFICATION

(11) **EP 2 387 970 B1**
(45) Date of publication and mention of the grant of the patent: **03.07.2013**
(21) Application number: 10305513.3
(22) Date of filing: 17.05.2010
(51) Int. Cl.: A61F 2/08

(54) **Endoprosthetic textile scaffold, especially for repairing rotator cuff tissue of human shoulder**
Endoprothetisches Textilgerüst, insbesondere zur Reparatur von Rotatorenmanschettengewebe einer menschlichen Schulter
Base textile endoprothétique, notamment pour la réparation de tissu de la coiffe du rotateur de l'épaule humaine

(43) Date of publication of application: 23.11.2011
(73) Proprietor: Tornier, Inc., Bloomington, MN 55437 (US)
(72) Inventor: Peterson, Dale, La Jolla CA 92037 (US); Mattern, Ralph, San Diego CA 92129 (US); Popper, Peter, Wilmington Delaware 19810 (US); Emmitt, Richard, Oldwick NJ 08858 (US); Rizik, Said, Salem NH 03079 (US); Ohashi, Kevin, Jamaica Plain MA 02130 (US); Ball, Robert J., West Olive Michigan 49460 (US)
(74) Representative: Grand, Guillaume

(56) References cited:
- EP-A1- 1 591 134
- WO-A2-02/067792
- US-A- 4 368 234
- US-A1- 2005 240 262
- US-A1- 2008 188 936

## Description

The present invention relatee to an endoprosthetic textile scaffold for repairing human tendons and ligaments.

The invention may be used in particular, but not exclusively, to repair the rotator cuff tissue in a human shoulder. Surgical repair of the rotator cuff tissue is usually accomplished by reattaching the tendons or the ligaments in apposition to the region of bone, from which it tore. Such a surgical repair Is performed as an open procedure or a fully arthroscopic procedure.

However, when direct reattachment is not possible, especially because of a large retraction of the tendon, the defect is filled by interposing an endoprosthesis. Such an endoprosthesis is also used to improve the strength of the repair.

It is known to make such an endoprosthesis from human or animal tissues. However, these biologic grafts have poor mechanical properties, which allows the repair to subsequently re-tear.

It is also known to use synthetic scaffolds as such an endoprosthesis. In particular, US-A-2007/0198087 describes a scaffold made of multiple layers of knits and non-woven fibers for rotator cuff repair. This scaffold is made of TephaFlex (registered mark), which is complex to manufacture and has limited mechanical properties. For its part, WO-A-2002/030324 describes textile configurations of scaffolds which are made by embroidery equipment. These textile configurations can be mechanically efficient but, firstly, they have to be implanted carefully because their mechanical properties are anisotropic and, secondly, they have predetermined shapes.

US-A-2008/0188936 discloses a scaffold according to the preamble of appended claim 1, that is to say a scaffold comprising one leno weave. Such a leno weave is strong and stiff along the warp or weft direction thereof but it collapses if pulled off-axis, eg at 45 degrees to the warp direction. The rather rectangular shaped spaces between the fibers narrow as the scaffold stretches. It requires almost no force to stretch the fabric off-axis until the spaces have collapsed. Thus this scaffold of US-A-2008/0188936 works for certain ligaments, but would not work well in tissues that sustain forces in off-axis directions such as the rotator cuff tendon.

An object of the present invention is to propose an endoprosthetic textile scaffold for repairing human tendons and ligaments, which combines great mechanical and healing properties and an case of use.

To this end, the subject of the invention is an endoprosthetic textile scaffold as defined in appended claim 1. Additional advantageous features of this scaffold are specified In dependent claims 2 to 15.

One of the ideas underlying the invention is to propose a bi-layer leno woven scaffold with its layers bound off-axis to each other. By that way, the mechanical properties of the scaffold are substantially isotropic, which enables surgeon to implant the scaffold in any desired orientation with respect to a tendon or ligament to be repaired. Besides, the woven structure of the scaffold is advantageously adapted to include, especially by impregnation, a delivery of biologic agents, in particular those biologic agents which encourage healing. Under these circumstances, the scaffold according to the invention may be preferably used to be sandwiched between a soft tissue, as a tendon or a ligament, and a bone to encourage healing of the interface between these two human tissues. For example, the scaffold may be used to repair the rotator cuff tissue in a human shoulder. Each of the leno weave of the scaffold has also the advantage of being cutable in arbitrary shapes, without unraveling or fraying. Besides, such a leno weave is easy to be manufactured and arranged in the scaffold according to the invention. Besides, in some embodiments of the present invention, the at least two leno weaves are made of fibers, especially monofilament fibers, which improve the human tissue growth into the scaffold. Furthermore, such fibers are preferably made of TephaFlex, which allows making resorbable monofilaments woven as a leno.

In addition, according to some embodiments of the invention, the at least two leno weaves of the scaffold are coated, especially to form a porous sponge, in particular a porous sponge made of collagen.

In practice, the scaffold according to the invention can be implanted using an open procedure or using minimal invasive arthroscopic surgical techniques.

Embodiments of the invention will be better understood from reading the description which will follow, which is given solely by way of example and with reference to the drawings in which:
- Figure 1 is a schematic exploded view of a scaffold according to an embodiment of the present invention;
- Figure 2 is a schematic elevation taken along II of figure 1;
- Figure 3 is a view similar to figure 2, showing only one of the weaves shown in figures 1 and 2;
- Figures 4 and 5 are views similar to figure 3, respectively showing variants of the aforesaid weave; and
- Figure 6 is a view similar to figure 2, showing a second embodiment of the present invention.

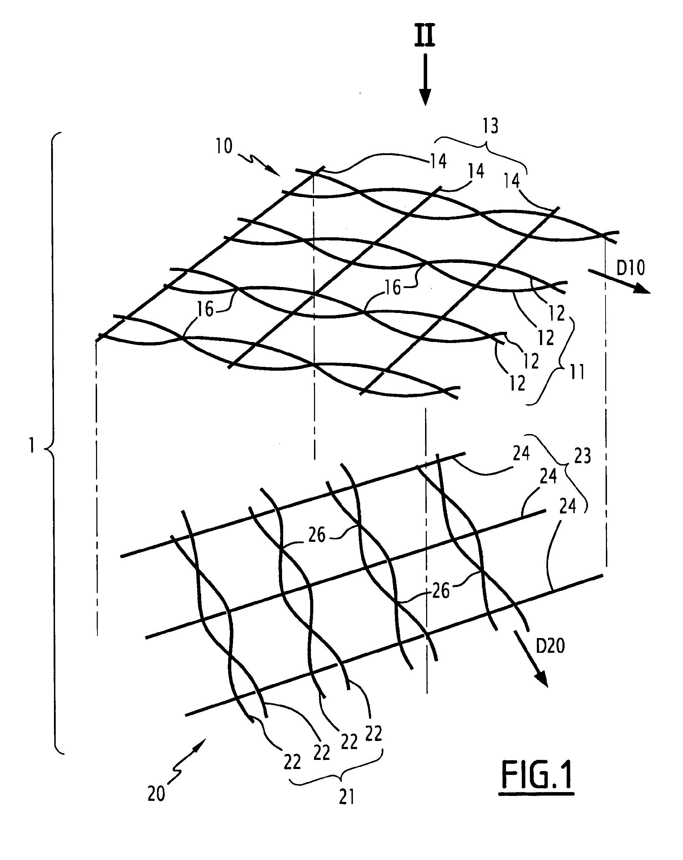

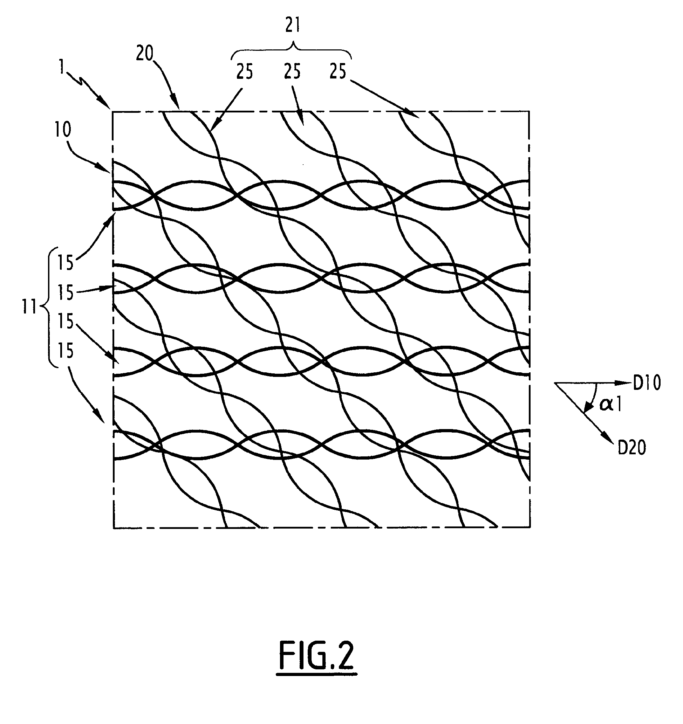

Figures 1 and 2 depict an endoprosthetic textile scaffold 1 comprising a first weave 10 and a second weave 20. In the shown embodiment, the two weaves 10 and 20 are identical More precisely, these two weaves 10 and 20 are substantially planar, that is to say that they correspond each to a bi-dimensional woven textile. Each weave 10, 20 includes a warp 11, 21 consisting of warp fibers 12, 22 oriented in length in a respective direction referenced D10, D20. Each weave 10, 20 further includes a weft 13, 23 consisting of weft fibers 14, 24.

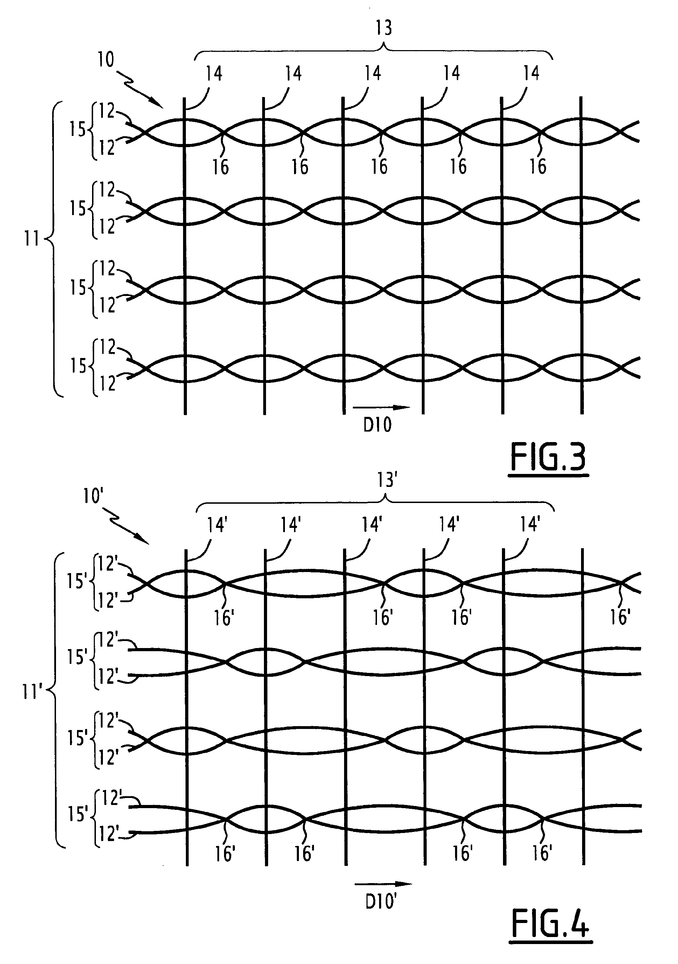

As better shown in figure 3 for weave 10, the weft fibers 14 are parallel to each other and run perpendicularly to direction D10, so that they interlace the warp fibers 12. In the same way, in weave 20, the weft fibers 24 are parallel to each other and run perpendicularly to direction D20, so that they interlace the warp fibers 22.

As best shown in figure 3 for weave 10, the warp fibers 12 of weave 10, respectively the warp fibers 22 of weave 20, are not parallel to each other but are associated by pairs 15, 25 of two warp fibers twisting around each other. The two warp fibers 12, 22 of each of these pairs 15, 25 cross at intersections 16, 26. These intersections 16, 26 are successive along the two warp fibers of each pair 15, 25, that is to say along direction D10, D20. Besides, each of these intersections 16, 26 between the two warp fibers of each of these pairs 15, 25 is located between two adjacent weft fibers 14, 24. In this way, the twisted warp fibers 12, 22 of each pair 15, 25 are locked in place with respect to weft 13, 23. In other words, the twisted warp fibers 12, 22 of each pair 15, 25 are prevented from slipping along the weft fibers 14, 24. As such, each weave 10, 20 does not unravel easily.

In practice, the textile woven structure, as described above, corresponds to a leno structure according to embodiments of the present invention. In other words, each weave 10, 20 may be a leno weave. As explained above, these leno weaves 10, 20 can be cut in arbitrary shapes without unraveling.

According to a non limiting example, the diameter of the fibers 12, 14, 22 and 24 is about 50 (in some cases 150) µm, and more than fifteen warp pairs 15, 25 are provided per inch, and more than twenty weft fibers 14, 24 are provided per inch.

As schematically shown in figures 1 and 2, the two weaves 10 and 20 are specifically positioned to each other in scaffold 1. More precisely, these two weaves 10 and 20 are arranged to be parallel superimposed. In other words, the respective planes containing weaves 10 and 20 are positioned parallel to each other. In this parallel configuration, the two directions D10 and D20 are positioned to be non parallel to each other. In other words, as shown in figure 2, in which weft 13, 23 of each weave 10, 20 is not depicted for clarity of the figure, the two directions D10 and D20 form between them an angle α1 that is different from 0° and 180°. It can be noted that, according to strict geometrical consideration, the aforesaid angle α1 is defined by the projections of directions D10 and D20 in a plane parallel to weaves 10 and 20.

Thus, as shown in figure 2, the warp fibers 12 of weave 10 run on the warp fibers 22 of weave 20, being biased with respect to the warp fibers 22 under angle α1. Of course, the same angulation is found between the weft fibers 14 of weave 10 and the weft fibers 24 of weave 20.

By that way, the mechanical properties of scaffold 1 are not anisotropic, especially in a main direction, but, on the contrary, these mechanical properties tend to be anisotropic, due at least in part to the respective mechanical effects provided by weave 10 and by weave 20. According to some embodiments of the present invention, angle α1 is about 45°. The aforesaid value of 45° includes the same value in either a clockwise direction or in a counterclockwise direction: in an angularly oriented plane, the aforesaid value is substantially equal to +45° and -45°.

The aforesaid mechanical properties for scaffold 1 may include tensile strength, stiffness, compliance, burst strength and/or suture pull-out force, according to embodiments of the present invention.

In addition to being different from 0° and 180°, angle α1 is different from 90° in order to avoid the weft fibers 24 of weave 20 running parallel to the warp fibers 12 of weave 10, according to embodiments of the present invention.

In order to maintain its mechanical properties in use, scaffold 1 is adapted to secure the weaves 10 and 20 with respect to each other, according to embodiments of the present invention. other. In other words, in the configuration shown in figure 2, the weaves 10 and 20 are bound to each other. In that way, forces applied on scaffold 1 are shared among all the weaves. In practice, different possibilities can be considered for such a binding.

A first solution is to laminate together the weaves 10 and 20 by interlacing these weaves on a loom: more precisely, after having woven one of the weaves 10 and 20, the other weave is woven on the first one, so that, due to the loom, the weft of this other weave is interlaced with the first one.

Another solution is to laminate together the weaves 10 and 20 by a plurality of stitches. For example, after having woven the two weaves independently from each other, an embroidery machine or a sewing machine may be used to sew stitches across the two weaves in order to bind the weaves together. These stitches may be sewn according to different stitching patterns selected, for example, from the group consisting of straight lines, "E" shapes, "W" or "zigzag" shapes, and the like. As such, mechanical isotropy of scaffold 1 is improved and stabilized by orienting these stitches transversally both to direction D10 and to direction D20, according to embodiments of the present invention.

According to one embodiment of the present invention, the weaves 10 and 20 are bound by interweaving these two weaves during their simultaneous manufacture. Such an interweaving may be performed on a multi-axis loom, although such looms are rare and complex.

In addition to the textile techniques for binding weaves 10 and 20 as discussed above, other techniques can be considered. For example, with an appropriate material constituting the fibers of the weaves 10 and 20, these two weaves can be ultrasonically bonded.

Furthermore, when the weaves 10 and 20 are secured to each other, these two weaves may be advantageously pressed against each other to increase the stability of their binding, to reduce the thickness of scaffold 1 and to improve the compliance of the scaffold.

According to an advantageous option, scaffold 1 is coated, which increases the mechanical stability of scaffold 1. According to embodiments of the present invention, the material used for such an external coating is selected from the group consisting of collagen, carboxymethylcellulose, cellulose, hyaluronic acid, chitosan, heparin, heparin sulphate, chondroitin sulphate, and fibrin or mixtures thereof.

In one embodiment, the aforesaid coating forms a porous sponge, especially after having lyophilized an appropriate material dispersion, such as a collagen dispersion. In that way, the external coating does not prevent biologic flows across scaffold 1. Such flows are desired for the scaffold in view of its use for repairing human tissues, as explained above.

Furthermore, in this context, scaffold 1 may advantageously include biologic agents: these agents may be, for example, impregnated in the weaves 10 and 20, for being released in tissues after implantation of scaffold,. Such biologic agents may be selected from the group consisting of growth factors, peptides, proteins, cells, cell extracts, platelet rich plasma, serum, serum extracts, bone marrow extracts, genes, DNA, RNA, siRNA, transcription factors, binding molecules, proteoglycans, carbohydrates, and/or chemokines, according to embodiments of the present invention.

According to one embodiment, the fibers 12, 14, 22 and 24 of the weaves 10 and 20 are made of polyhydroxyalkanoate, especially of TephaFlex (registered mark) which corresponds to poly-4-hydroxybutyrate.

Based on the disclosure provided herein, one of ordinary skill in the art will appreciate that other biocompatible materials, especially resorbable polymers, may be used. In particular, materials suitable for implantation in the body may be used, that is to say biocompatible materials, without prions or infectious agents, and with low levels of pyrogens or other inflammatory agents.

In practice, each of the fibers 12, 14, 22, 24 may be a monofilament. Monofilaments maintain space or pores between their intersections which encourages bone and soft tissue growth into scaffold 1. In addition or in substitution of such monofilaments, multifilaments may be used for forming the aforesaid fibers; in that way, the binding capacity of the weaves 10 and 20 is increased and can be sufficient even without an applied coating. When a multiplicity of such multifilaments are used, the external surfaces of scaffold 1 achieve a felt effect, which can be useful for specific implantations, according to embodiments of the present invention.

Leno weave 10 shown in figure 3 is not the only one woven structure that can be considered for each leno weave of scaffold 1. Figure 4 depicts a variant for weave 10, which is referenced 10'. Contrary to weave 10, in which only one weft fiber 14 is systematically interposed along direction D10, between two successive intersections 16 of the two-warp fibers 12 of each of pairs 15, two successive intersections 16' between the two warp fibers 12' of pairs 15' constituting warp 11' of weave 10' are alternatively separated by one of the fibers 14' of weft 13' and by two of these weft fibers. In this way, the packing of the fibers in weave 10' may be increased.

As shown by figure 4, for each of the pairs 15', two successive intersections 16', which are separated by two weft fibers 14', are positioned along direction D10' of weave 10', in a shifted manner with respect to the two adjacent pairs 15'. In other words, the intersections 16' (where the warp fibers 12' cross) alternate in direction D10', which permits denser packing of the warp pairs 15'.

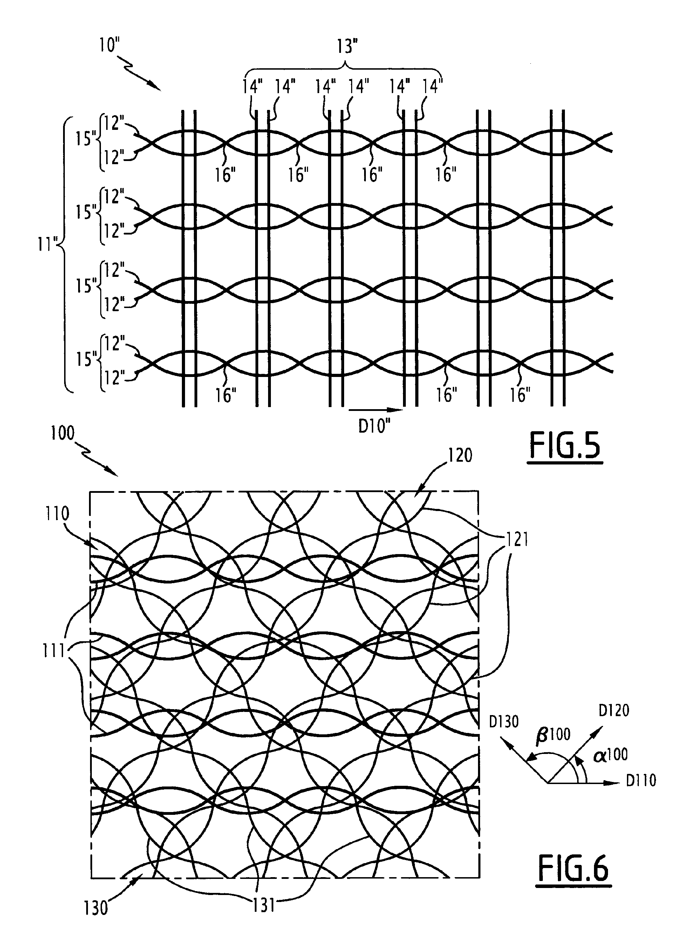

Figure 5 depicts another variant for weave 10, which is referenced 10". Contrary to weave 10', two weft fibers 14" of weft 13" are systematically interposed between two successive intersections 16" between the two warp fibers 12" of each of the warp pairs 15" constituting warp 11" of leno weave 10". In this way, the packing of the weft fibers 14" is permitted to be denser.

Figure 6 depicts a scaffold 100, which essentially differs from scaffold 1 by the number of its weaves: scaffold 100 comprises three weaves 110, 120 and 130, according to embodiments of the present invention. Taken individually, these weaves 110, 120 and 130 may be identical and may each have the same structure of weave 10 or 20. In particular, each of these weaves 110, 120 and 130 includes both a warp 111, 121, 131, oriented in its respective direction D110, D120, D130, and a weft, not drawn in figure 6 for clarity reasons, oriented perpendicularly to the aforesaid direction D110, D120, D130.

The three weaves 110, 120 and 130 are superimposed in a parallel manner and are bound to each other so that any one of the directions D110, D120 and D130 is non-parallel to the others. In other words, as indicated in figure 6, an angle α100 is formed between the directions D110 and D120 of the weaves 110 and 120, this angle α100 being different from 0° and 180°. And an angle β100 is formed between the directions D110 and D130 of the weaves 110 and 130, this angle β100 being different from 0°, 180° and α100.

According to a preferred embodiment, the angles between the three directions D110, D120 and D130 are regularly or uniformly distributed in the plane of parallelism between the weaves 110, 120 and 130 of scaffold 100. In other words, in an angularly oriented plane, angle α100 has a value of about +60° and angle β100 has a value of about +120°, according to embodiments of the present invention.

Suitable structures can also be made by interspersing small zones of leno weave with plain weave to increase the density and strength of the scaffold, by laminating leno woven layers with plain weave layers, or by weaving monofilaments in a plain weave intermixed with multifilament yarns woven in a leno manner to give higher strength, maintain porosity, and still produce stable scaffolds that can be trimmed to shape, according to embodiments of the present invention.

According to not shown embodiments of the invention, the weft of one or more of the at least two weaves of the scaffold 1 or 100 is not oriented in a direction directly perpendicular to the corresponding warp. In other words, in one or more of these weaves, the warp and the weft may be woven with a relative angle that is different from 90°.

In practice, each of the various arrangements and variants which have been described for scaffold 1 may be implemented for scaffold 100, according to embodiments of the present invention.

More generally, other embodiments of the invention are possible, including by recombining the various elements disclosed herein in different or alternative combinations. Although the above description contains many specifics, these should not be considered as limiting the scope of the invention as defined by the appended claims, but as merely providing illustrations of some of the embodiments of this invention.

## Claims

1. An endoprosthetic textile scaffold (1; 100) for repairing a human tendon or ligament, comprisinga first leno weave (10; 110), wich is substantially planar and which includes a warp (11; 111), oriented in a first direction (D10: D110), and a weft (13), **characterized in that** the scaffold further comprises a second leno weave (20; 120) which is substantially planar and which includes a warp (21), oriented in a second direction (D20; D120), and a weft (23), the second leno weave being arranged and bound to the first leno so that the first and second leno weaves are superimposed in a parallel manner, with the first direction being non-parallel to the second direction.

2. The scaffold according to claim 1, **characterized in that** the first (D10; D110) and second (D20) directions define between them an angle (α1; α100) that is different from 0°, 90° and 180°,

3. The scaffold according to claim 1 or claim 2, **characterized in that**, in an angularly oriented plane, the angle (α1; α100) between the first (D10) and second (D20) directions has a value of about +45° or about -45°.

4. The scaffold according to any one of preceding claims, **characterized in that** the scaffold (100) further comprises a third leno weave (130), which Is substantially planar and which includes a warp (131), oriented in a third direction (D130), and a weft,
and **in that** the third leno weave is arranged and bound to the first (110) and second (120) leno weaves so that the first, second and third leno weaves are superimposed in a parallel manner with the third direction (D130) being non parallel both to the first direction (D110) and to the second direction (D120).

5. The scaffold according to claim 4, **characterized in that**, in an angularly oriented plane, the angle (α100) between the first (D110) and second (D120) directions has a value of about +60° and the angle (β100) between the first (D100) and third (D130) directions has a value of about +120°.

6. The scaffold according to any one of preceding claims, **characterized in that** the warp (11; 11'; 11") of each of the first (10; 10'; 10") and second and, if present, third leno weaves consists of a plurality of warp fibers (12; 12'; 12") running along, respectively, the first (D10; D10'; D10"), second or third direction,
**in that** the weft (13; 13'; 13") of each of the first and second and, if present, third leno weaves consists of a plurality of parallel weft fibers (14; 14'; 14") which run perpendicularly to respectively the first, second or third direction and which interlace the warp fibers of their corresponding leno weave,
and **in that** the warp fibers (12; 12'; 12') of each of the first (10; 10'; 10") and second and, if present, third leno weaves are associated by pairs (15; 15'; 16") of two warp fibers twisting around each other so that the two warp fibers of each of said pairs cross at intersections (16 ; 16' ; 16"), which are arranged in succession along, respectively, the first (D10: D10'; D10"), second or third direction and which are each located between two adjacent weft fibers (14; 14'; 14").

7. The scaffold according to claim 6, **characterized in that** only one weft fiber (14; 14') is, systematically or alternatively, interposed between two successive intersections (18, 16') between the two warp fibers (12; 12') of each of said pairs (16; 15').

8. The scaffold according to claim 6 or claim 7, **characterized In that** at least two weft fibers (14'; 14") are, systematically or alternatively, interposed between two successive intersections (18'; 16") between the two warp fibers (12'; 12") of each of said pairs (15'; 15").

9. The scaffold according to any one of claims 6 to 9B **characterized in that** at least some of the fibers (12; 14; 12; 14'; 12"; 14"; 22; 24) respectively consist of monofilaments.

10. The scaffold according to claim 9, **characterized in that** said monofilaments are larger than 50 µm In diameter.

11. The scaffold according to any one of claims 6 to 10, **characterized in that** at least some of the fibers (12; 14; 12'; 14'; 12"; 14"; 22; 24) respectively consist of multifilaments.

12. The scaffold according to any one of claims 6 to 11, **characterized in that** the fibers (12; 14; 12'; 14'; 12"; 14"; 22; 24) are made of polyhydroxyalkanoate.

13. The scaffold according to any one of the preceding claims, **characterized in that** the first leno weave (10; 110) and the second leno weave (20; 120) and, if present, the third leno weave (130) are bound to each other by being laminated together,
and **in that** these leno weaves are laminated by one or more of:
- Interlacing, especially on a loom, the weft of one of the leno weaves with the other leno weave that in beforehand woven, and
- a plurality of stitches which are preferable oriented transversely to both the first (D10; D110), second (D20; D120) and third (D130) directions and which are especially sewn with an embroidery or sewing machine.

14. The scaffold according to any one of claims 1 to 12, **characterized in that** the first leno weave (10; 110) and the leno second weave (20; 120) and, if present, the third leno weave (130) are bound to each other by being interwoven during a simultaneous weaving.

15. The scaffold according to any of the preceding claims, **characterized in that** the scaffold (1; 100) further comprises an external coating, especially forming a porous sponge.

## Patentansprüche

1. Endoprothetisches Textilgerüst (1; 100) zum Reparieren einer menschlichen Sehne oder eines menschlichen Bandes, wobei das Gerüst ein erstes Gazegewebe (10; 110) aufweist, das im wesentlichen plan ist und das eine Kette (11; 111), die in einer ersten Richtung (D10; D110) orientiert ist, und einen Schuss (13) aufweist,
**dadurch gekennzeichnet,**
**dass** das Gerüst ferner ein zweites Gazegewebe (20; 120) aufweist, das im wesentlichen plan ist und das eine Kette (21), die in einer zweiten Richtung (D20; D120) orientiert ist, und einen Schuss (23) aufweist, wobei das zweite Gazegewebe derart angeordnet und mit dem ersten Gazegewebe verbunden ist, dass das erste und das zweite Gazegewebe in einer parallelen Weise einander überlagert sind, wobei die erste Richtung nicht parallel zu der zweiten Richtung verläuft.

2. Gerüst nach Anspruch 1,
**dadurch gekennzeichnet,**
**dass** die erste Richtung (D10; D110) und die zweite Richtung (D20) zwischeneinander einen Winkel (α1; α100) bilden, der von 0°, 90° und 180° verschieden ist.

3. Gerüst nach Anspruch 1 oder 2,
**dadurch gekennzeichnet,**
**dass** in einer unter einem Winkel orientierten Ebene der Winkel (α1; α100) zwischen der ersten Richtung (D10) und der zweiten Richtung (D20) einen Wert von etwa +45° oder etwa -45° besitzt.

4. Gerüst nach einem der vorhergehenden Ansprüche,
**dadurch gekennzeichnet,**
**dass** das Gerüst (100) ferner ein drittes Gazegewebe (130) aufweist, das im wesentlichen plan ist und das eine Kette (131), die in einer dritten Richtung (D130) orientiert ist, und einen Schuss aufweist,
und **dass** das dritte Gazegewebe derart angeordnet und mit dem ersten Gazegewebe (110) und dem zweiten Gazegewebe (120) verbunden ist, dass das erste, das zweite und das dritte Gazegewebe in einer parallelen Weise einander überlagert sind, wobei die dritte Richtung (D 130) nicht parallel zu der ersten Richtung (D110) und der zweiten Richtung (D120) verläuft.

5. Gerüst nach Anspruch 4,
**dadurch gekennzeichnet,**
**dass** in einer unter einem Winkel orientierten Ebene der Winkel (α100) zwischen der ersten Richtung (D110) und der zweiten Richtung (D120) einen Wert von etwa +60° besitzt und der Winkel (β100) zwischen der ersten Richtung (D100) und der dritten Richtung (D130) einen Wert von etwa +120° besitzt.

6. Gerüst nach einem der vorhergehenden Ansprüche,
**dadurch gekennzeichnet,**
**dass** die Kette (11; 11'; 11") von jedem von dem ersten Gazegewebe (10; 10'; 10") und dem zweiten Gazegewebe und, falls vorhanden, dem dritten Gazegewebe aus einer Vielzahl von Kettfasern (12; 12'; 12") besteht, die jeweils längs der ersten Richtung (D10; D10'; D10"), der zweiten Richtung oder der dritten Richtung verlaufen,
**dass** der Schuss (13; 13'; 13") von jedem von dem ersten Gazegewebe und dem zweiten Gazegewebe und, falls vorhanden, von dem dritten Gazegewebe aus einer Vielzahl von parallelen Schussfasern (14; 14'; 14") besteht, die senkrecht zu der jeweiligen ersten, zweiten oder dritten Richtung verlaufen und die mit den Kettfasern der entsprechenden Gazegewebe verschlungen sind,
und **dass** die Kettfasern (12; 12'; 12") von jedem von dem ersten Gazegewebe (10; 10'; 10") und dem zweiten Gewebe und, falls vorhanden, dem dritten Gazegewebe paarweise (15; 15'; 15") von zwei Kettfasern zugeordnet sind, die umeinander verzwirnt sind, sodass die beiden Kettfasern von jedem von den Paaren an Kreuzungen (16; 16'; 16") einander kreuzen, die der Reihe nach längs der jeweiligen ersten Richtung (D10; D10'; D10"), der zweiten Richtung oder der dritten Richtung angeordnet sind und die jeweils zwischen zwei benachbarten Schussfasern (14; 14'; 14") angeordnet sind.

7. Gerüst nach Anspruch 6,
**dadurch gekennzeichnet,**
**dass** nur eine Schussfaser (14; 14') systematisch oder abwechselnd zwischen zwei aufeinanderfolgende Kreuzungen (16; 16') zwischen den beiden Kettfasern (12; 12') von jedem der Paare (15; 15') dazwischengesetzt sind.

8. Gerüst nach Anspruch 6 oder 7,
**dadurch gekennzeichnet,**
**dass** zumindest zwei Schussfasern (14'; 14") systematisch oder abwechselnd zwischen zwei aufeinanderfolgenden Kreuzungen (16'; 16") zwischen den beiden Kettfasern (12'; 12") von jedem der Paare (15'; 15 ") dazwischengesetzt sind.

9. Gerüst nach einem der Ansprüche 6 bis 8,
**dadurch gekennzeichnet,**
**dass** zumindest einige der Fasern (12; 14; 12'; 14'; 12"; 14"; 22; 24) jeweils aus Monofilamenten besteht.

10. Gerüst nach Anspruch 9,
**dadurch gekennzeichnet,**
**dass** die Monofilamente einen Durchmesser besitzen, der größer als 50 µm ist.

11. Gerüst nach einem der Ansprüche 6 bis 10,
**dadurch gekennzeichnet,**
**dass** zumindest einige der Fasern (12; 14; 12'; 14'; 12"; 14"; 22; 24) jeweils aus Multifilamenten bestehen.

12. Gerüst nach einem der Ansprüche 6 bis 11,
**dadurch gekennzeichnet,**
**dass** die Fasern (12; 14; 12'; 14'; 12"; 14"; 22; 24) aus Polyhydroxyalkanoat bestehen.

13. Gerüst nach einem der vorhergehenden Ansprüche,
**dadurch gekennzeichnet,**
**dass** das erste Gazegewebe (10; 110) und das zweite Gazegewebe (20; 120) und, falls vorhanden, das dritte Gazegewebe (130) dadurch miteinander verbunden sind, dass sie zusammenlaminiert sind,
und **dass** diese Gazegewebe durch eine oder mehrere der folgenden Maßnahmen laminiert sind:
- Verweben, insbesondere auf einem Webstuhl, des Schusses von einem der Gazegewebe mit dem anderen Gazegewebe, das vorher gewebt worden ist, und
- eine Vielzahl von Stichen, die vorzugsweise quer zu sowohl der ersten Richtung (D10; D110), der zweiten Richtung (D20; D120) und der dritten Richtung (130) orientiert sind und die insbesondere mit einer Stickereimaschine oder Nähmaschine angebracht werden.

14. Gerüst nach einem der Ansprüche 1 bis 12,
**dadurch gekennzeichnet,**
**dass** das erste Gazegewebe (10; 110) und das zweite Gazegewebe (20; 120) und, falls vorhanden, das dritte Gazegewebe (130) dadurch miteinander verbunden werden, dass sie während eines gleichzeitigen Webvorganges miteinander verwebt werden.

15. Gerüst nach einem der vorhergehenden Ansprüche,
**dadurch gekennzeichnet,**
**dass** das Gerüst (1; 100) ferner eine äußere Beschichtung aufweist, die insbesondere einen porösen Schwamm bildet.

## Revendications

1. Base textile endoprothétique (1 ; 100) pour réparer un tendon ou un ligament humain, comprenant une première armure gaze (10 ; 110) qui est sensiblement plane et qui comprend une chaîne (11 ; 111) orientée dans une première direction (D10 ; D110) et une trame (13), **caractérisée en ce que** la base comprend une deuxième armure gaze (20 ; 120) qui est sensiblement plane et qui comprend une chaîne (21) orientée dans une deuxième direction (D20 ; D120), et une trame (23), la deuxième armure gaze étant agencée et reliée à la première armure gaze de sorte que les première et deuxième armures gazes sont superposées d'une manière parallèle, avec la première direction qui n'est pas parallèle à la deuxième direction.

2. Base selon la revendication 1, **caractérisée en ce que** la première (D10 ; D110) et la deuxième (D20) direction définissent un angle (α1; α100) entre elles qui est différent de 0°, 90° et 180°.

3. Base selon la revendication 1 ou la revendication 2, **caractérisée en ce que**, dans un plan orienté de manière angulaire, l'angle (α1; α100) entre les première (D10) et deuxième (D20) directions a une valeur d'environ +45° ou environ -45°.

4. Base selon l'une quelconque des revendications précédentes, **caractérisée en ce que** la base (100) comprend un outre une troisième armure gaze (130) qui est sensiblement plane et qui comprend une chaîne (131) orientée dans une troisième direction (D130), et une trame,
et **en ce que** la troisième armure gaze est agencée et reliée à la première (110) et à la deuxième (120) armure gaze de sorte que les premières, deuxième et troisième armures gazes sont superposées d'une manière parallèle à la troisième direction (D130) en étant non parallèle à la première direction (D110) et à la deuxième direction (D120).

5. Base selon la revendication 4, **caractérisée en ce que**, dans un plan orienté de manière angulaire, l'angle (α100) entre les première (D110) et seconde (D120) directions a une valeur d'environ +60° et l'angle (β100) entre les première (D100) et troisième (D130) directions a une valeur d'environ +120°.

6. Base selon l'une quelconque des revendications précédentes, **caractérisée en ce que** la chaîne (11; 11'; 11") de chacune des première (10; 10'; 10") et deuxième et, si elle existe, troisième armures gazes se compose d'une pluralité de fibres de chaîne (12; 12'; 12") s'étendant respectivement le long des première (D10; D10'; D10"), deuxième ou troisième directions,
**en ce que** la trame (13; 13'; 13") de chacune les première et deuxième et, si elle existe, troisième armures gazes se compose d'une pluralité de fibres de trame parallèles (14; 14'; 14") qui s'étendent perpendiculairement, respectivement, aux première, deuxième ou troisième directions et qui entrelacent les fibres de chaîne de leur armure gaze correspondante,
et **en ce que** les fibres de chaîne (12; 12'; 12") de chacune des première (10; 10'; 10") et deuxième et, si elle existe, troisième armures gazes sont associées par des paires (15; 15'; 15") de deux fibres de chaîne qui se torsadent l'une autour de l'autre de sorte que les deux fibres de chaîne de chacune desdites paires se croisent aux intersections (16; 16'; 16") qui sont agencées en succession respectivement le long de la première (D10; D10'; D10"), la deuxième ou la troisième direction et qui sont chacune positionnées entre deux fibres de trame (14; 14'; 14") adjacentes.

7. Base selon la revendication 6, **caractérisée en ce que** seule une fibre de trame (14; 14') est, systématiquement ou de manière alternée, intercalée entre deux intersections successives (16, 16') entre les deux fibres de chaîne (12; 12') de chacune desdites paires (15; 15').

8. Base selon la revendication 6 ou la revendication 7, **caractérisée en ce qu'**au moins deux fibres de trame (14'; 14") sont, systématiquement ou de manière alternée, intercalées entre deux intersections (16'; 16") successives entre les deux fibres de chaîne (12'; 12") de chacune desdites paires (15'; 15").

9. Base selon l'une quelconque des revendications 6 à 8, **caractérisée en ce qu'**au moins certaines des fibres (12; 14; 12'; 14'; 12"; 14"; 22; 24) se composent respectivement de monofilaments.

10. Base selon la revendication 9, **caractérisée en ce que** lesdits monofilaments ont un diamètre supérieur à 50 µm.

11. Base selon l'une quelconque des revendications 6 à 10, **caractérisée en ce qu'**au moins certaines des fibres (12; 14; 12'; 14'; 12"; 14"; 22; 24) se composent respectivement de multifilaments.

12. Base selon l'une quelconque des revendications 6 à 11, **caractérisée en ce que** les fibres (12; 14; 12'; 14'; 12"; 14"; 22; 24) sont réalisées à partir de polyhydroxyalcanoate.

13. Base selon l'une quelconque des revendications précédentes, **caractérisée en ce que** la première armure gaze (10; 110) et la deuxième armure gaze (20; 120) et, si elle existe, les troisièmes armures gazes (130) sont reliées entre elles en étant stratifiées ensemble, et **en ce que** ces armures gazes sont stratifiées selon un ou plusieurs des éléments suivants:
entrelacer, en particulier sur un métier à tisser, la trame de l'une des armures gazes avec l'autre armure gaze qui a été tissée auparavant, et
une pluralité de points qui sont de préférence orientés de manière transversale à la fois par rapport aux première (D10; D110), deuxième (D20; D120) et troisième (D130) directions et qui sont en particulier cousus avec une machine à broder ou à coudre.

14. Base selon l'une quelconque des revendications 1 à 12, **caractérisée en ce que** la première armure gaze (10; 110) et la deuxième armure gaze (20; 120) et, si elle existe, la troisième armure gaze (130) sont reliées entre elles en étant tissées pendant un tissage simultané.

15. Base selon l'une quelconque des revendications précédentes, **caractérisée en ce que** la base (1; 100) comprend en outre un revêtement externe, en particulier formant une éponge poreuse.
